# EUROPEAN PATENT SPECIFICATION

(11) **EP 3 320 956 B1**
(45) Date of publication and mention of the grant of the patent: **01.01.2020**
(21) Application number: 17199921.2
(22) Date of filing: 03.11.2017
(51) Int. Cl.: A63B 33/00, B65D 63/16

(54) **BELT LENGTH ADJUSTER**
GÜRTELLÄNGENEINSTELLUNG
RÉGLEUR DE LONGUEUR DE COURROIE

(30) Priority: 11.11.2016 JP 2016220257
(43) Date of publication of application: 16.05.2018
(73) Proprietor: Yamamoto Kogaku Co., Ltd., Higashiosaka-shi Osaka (JP)
(72) Inventor: ASADA, Masataka, Higashiosaka-shi, Osaka (JP); OOBA, Tomoyuki, Higashiosaka-shi, Osaka (JP)
(74) Representative: SSM Sandmair

(56) References cited:
- WO-A1-2004/020046
- CH-A- 10 880
- US-A- 200 658
- US-A- 206 179
- US-A- 2 889 599
- US-A- 3 377 666
- US-A- 3 407 452

## Description

### BACKGROUND OF THE INVENTION

### Field of the Invention

The present invention relates to a belt length adjuster used for belts of facial accessories such as goggles, glasses, and masks or belts of clothing and accessories such as bags, gloves, and clothes, and more particularly, to a belt length adjuster suitable for a belt of swimming goggles.

### Description of the Related Art

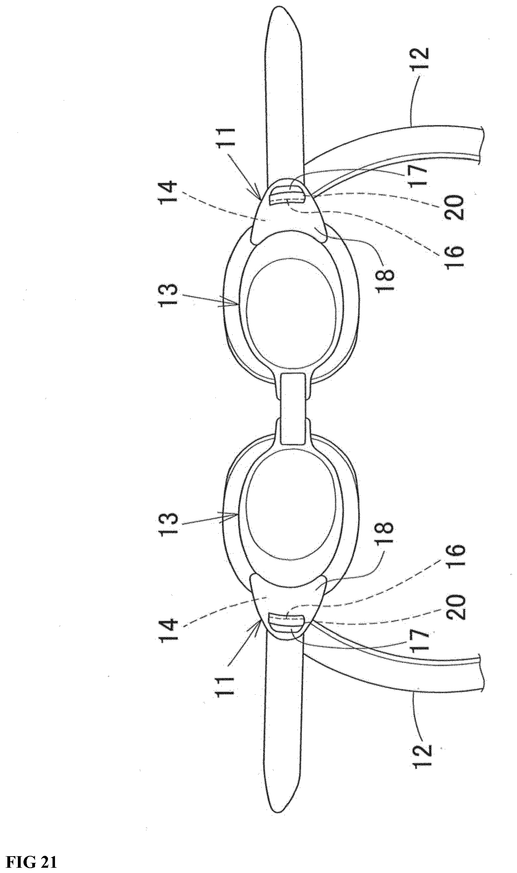

Conventionally, as this type of belt length adjuster, for example, there is a belt length adjuster used for a belt of swimming goggles such as shown in FIGS. 21 to 23 (Japanese Patent Laid-Open No. 2003-236018).

This belt length adjuster is configured such that belt connectors 11 each having a face plate 18 and a connecting piece 20 are placed on front sides of left and right outer end portions 14 of a goggle body and that a belt 12 passed through belt insertion holes 15 in the outer end portions 14 is wound around the connecting pieces 20 and is then pulled out through the belt insertion holes 15. That is, belt passage slots 16 and 17 are formed in an outer end portion of each face plate 18 and a long connecting piece 20 elongated in a right-left direction is formed by extending an intermediate portion between the belt passage slots 16 and 17 inward. Then, when the belt connector 11 is attached to the outer end portion 14, the belt 12 wound around the connecting piece 20 is pinched by the connecting piece 20 and an outer constituent wall 15a of the belt insertion hole 15.

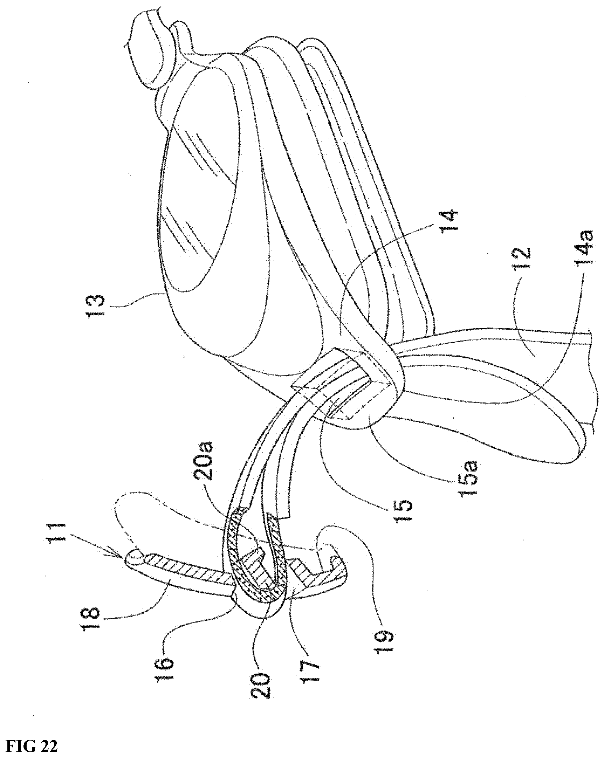

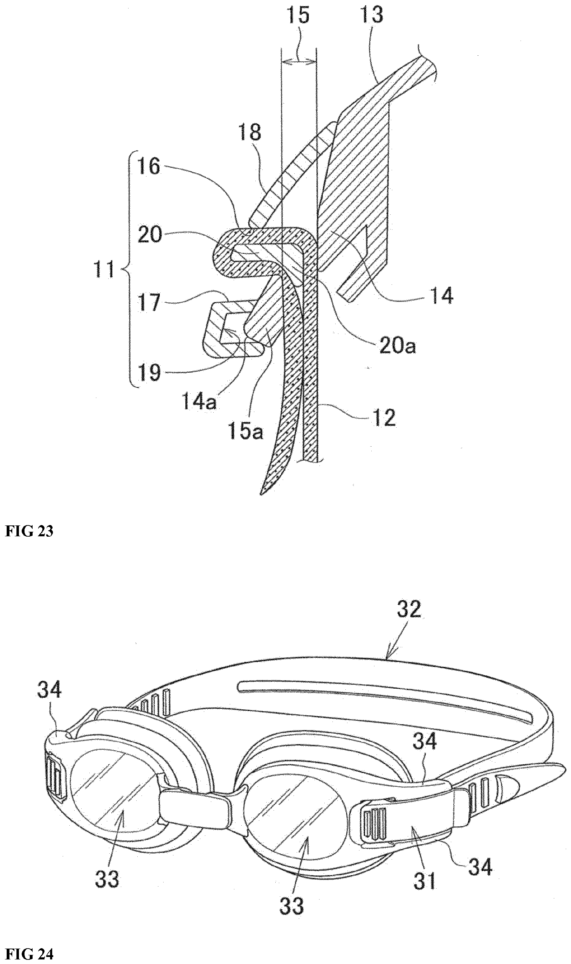

With the belt length adjuster which is configured in this way, to attach the belt 12 to an eye cup 13, first, as shown in FIG. 22, one end of the belt 12 is passed through the belt insertion hole 15 in the outer end portion 14 on one side of an eye cup 13, passed through the belt passage slots 16 and 17 in the belt connector 11 removed from the eye cup 13, and then passed through the belt insertion hole 15 again. Next, the belt 12 is pulled strongly until a relationship between the belt connector 11 and the outer end portion 14 shown in FIG. 23 is satisfied, and the belt connector 11 is attached to the eye cup 13. Regarding another end of the belt 12, a similar operation is repeated to attach the belt connector 11 to the eye cup 13.

When the above operations are finished, a fitting recess 19 of the face plate 18 has been fitted over an outside edge 14a of the outer end portion 14 and the outer constituent wall 15a of the belt insertion hole 15 has been pinched between a constituent wall of the fitting recess 19 and a bent portion 20a of the connecting piece 20. Then, the belt 12 wound around the connecting piece 20 is kept pinched between the outer constituent wall 15a of the belt insertion hole 15 and the bent portion 20a of the connecting piece 20, and the belt 12 does not slip over to the connecting piece 20. Therefore, with the belt length adjuster, the belt 12 does not loosen inadvertently.

Furthermore, as this type of belt length adjuster, for example, there is a belt length adjuster used for a belt of swimming goggles such as shown in FIGS. 24 to 26 (Japanese Patent Laid-Open No. 2012-139360).

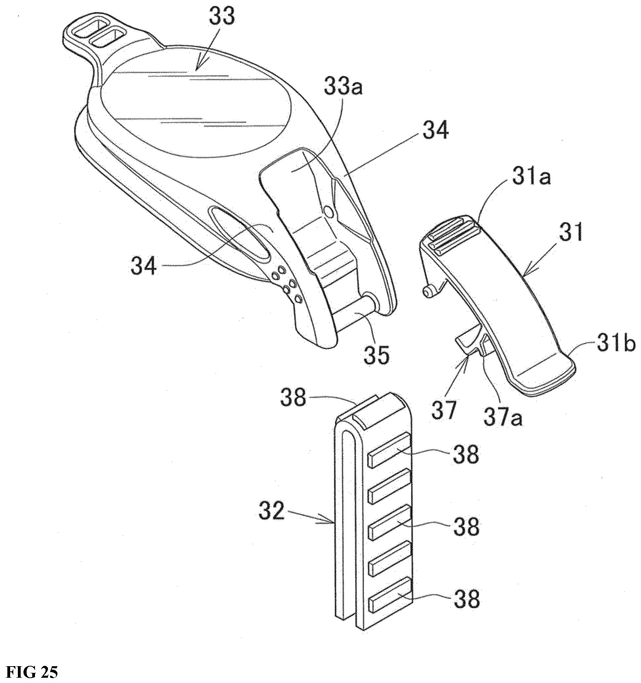

This belt length adjuster is configured such that a belt 32 is attached to an eye cup 33 through an operating member 31. As shown in FIG. 25, a pressing surface 33a is formed in an end portion of an eye cup 33 and a pair of support arms 34 are provided integrally at required spacing on the pressing surface 33a. A spindle 35 used to wind the belt 32 is provided between tips of the support arms 34. Furthermore, the operating member 31 is pivotally mounted between the support arms 34.

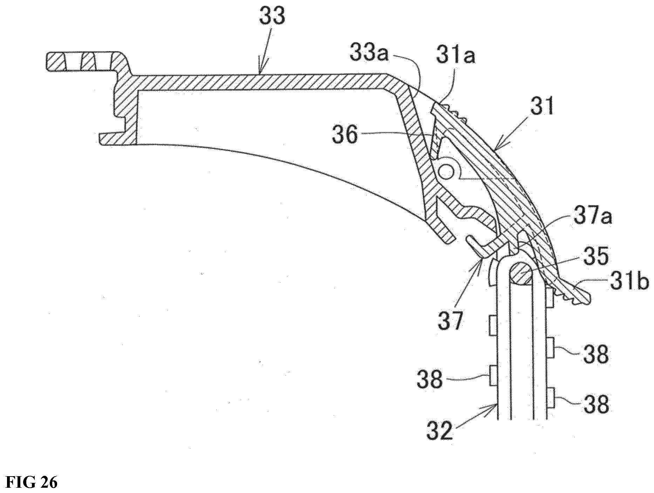

An inner end of the operating member 31 is a pressing operation portion 31a and an outer end is a pulling operating portion 31b. Facing the pressing operation portion 31a, an elastic strip 36 (see FIG. 26) configured to elastically deform by pressing the pressing surface 33a of the eye cup 33 is provided on the operating member 31 and an engagement body 37 having an engaging claw 37a is provided in an intermediate portion between the pressing operation portion 31a and the pulling operating portion 31b. The engaging claw 37a is disengageably engaged with any of plural protrusions 38 provided in a longitudinal direction on a surface side of the belt 32 turning back after being wound around the spindle 35.

Being configured as described above, the belt length adjuster makes the operating member 31 easy to operate when the swimming goggles are worn, allows the belt 32 to be loosened by either a pushing operation or pulling operation and thereby provides great convenience.

However, with the belt length adjuster which is described in Japanese Patent Laid-Open No. 2003-236018, to adjust belt length, the belt connector 11 has to be removed from the eye cup 13 as shown in FIG. 22. There is a problem in that it is difficult to remove the belt connector 11 from the eye cup 13 with the swimming goggles worn and that the belt length cannot be adjusted properly without removing the swimming goggles.

Furthermore, with the belt length adjuster which is described in Japanese Patent Laid-Open No. 2012-139360, although the length of the belt 32 can be adjusted with the swimming goggles worn, because a structure of the end portion of the eye cup 33 becomes complicated, there is a problem of reduced production efficiency, resulting in increased production costs. Also, the belt length adjuster, in which the end portion of the eye cup 33 is bulky and protrusions 38 need to be provided on the surface of the belt 32 as well, is disadvantageous in terms of increased fluid resistance during competitive swimming and the like.

The document CH 10880 A relates to a belt length adjuster comprising first and second belt pinching rods articulating around a common axis in their middle in a fixed angle between the first and second pinching rods.

The document US 2,889,599 A relates to a strap buckle composed of two parts, namely a closed loop and a U-bolt, both formed of steel bar. The loop is rectangular and has a central opening, while the U-bolt has a first straight leg and a second leg with a locking tip projecting outwardly from the leg at right angle to the longitudinal dimension and away from the plane of the U-bolt at an angle of about 60 °.

The document US 3,377,666 relates to a buckle for flexible strapping which buckle is formed from a single piece of wire. The wire is shaped into a first generally U-shaped portion, a second generally U-shaped portion and an intermediate wire portion. The first portion includes a distal leg, a shorter proximal leg and an intermediate bight. The second portion also includes a distal leg, a shorter proximal leg and an intermediate bight. The intermediate portion connects the proximal legs of the first and second portions.

Thus, the present invention has been made to provide a belt length adjuster which makes it easy to carry out a belt length adjustment operation with eyewear or the like worn, has a simple structure, allows production costs to be reduced as well, and has the advantage of causing low fluid resistance during competitive swimming and the like because of compactness when used for swimming goggles.

### SUMMARY OF THE INVENTION

The present invention provides a belt length adjuster as claimed in claim 1.

The belt length adjuster of the present invention comprises a belt pinching frame F, the belt pinching frame F comprising a first pinching frame 1 and a second pinching frame 2 pivotally intersecting each other, a crossing angle α between the first and second pinching frames 1 and 2 being able to be opened and closed, wherein a first end of a belt B is inserted into a first end of the belt pinching frame F and a second end of the belt B is inserted into a second end of the belt pinching frame F, the first end and the second end of the belt B are pinched between the first and second pinching frames 1 and 2 when the crossing angle α is fully closed, and the first end and the second end of the belt B are released from pinching when the crossing angle α is fully opened; wherein the first pinching frame 1 includes a lower pinching frame body 3 on one side and an upper pinching frame body 5 on another side across a pivot portion 4; the lower pinching frame body 3 includes opposite side frame portions 3a and 3b and an outer frame portion 3c; a first insertion space S₁ for a belt B is provided on an inner side of the outer frame portion 3c; the upper pinching frame body 5 includes opposite side frame portions 5a and 5b, an outer frame portion 5c, and a middle frame portion 5d; a second insertion space S₂ for the belt B is provided on an inner side of the middle frame portion 5d; a third insertion space S₃ for the belt B is provided between the outer frame portion 5c and the middle frame portion 5d; the second pinching frame 2 includes a lower pinching frame body 6 on one side and an upper pinching frame body 8 on another side across a pivot portion 7; the lower pinching frame body 6 includes opposite side frame portions 6a and 6b and an outer frame portion 6c; the first insertion space S₁ for the belt B is provided on an inner side of the outer frame portion 6c; the upper pinching frame body 8 includes opposite side frame portions 8a and 8b, an outer frame portion 8c, and a middle frame portion 8d; the second insertion space S₂ for the belt B is provided on an inner side of the middle frame portion 8d; and the third insertion space S₃ for the belt B is provided between the outer frame portion 8c and the middle frame portion 8d.

In the belt length adjuster according to the present invention, the first end of the belt B is pinched between the outer frame portion 3c of the first pinching frame 1 and the outer frame portion 8c of the second pinching frame 2; and the second end of the belt B is pinched between the outer frame portion 5c of the first pinching frame 1 and the outer frame portion 6c of the second pinching frame 2.

In the belt length adjuster according to the present invention, the crossing angle α is set to 10 to 90 degrees.

In the belt length adjuster according to the present invention, the belt B is made of an elastic synthetic resin or synthetic rubber with an elasticity of 25 to 65 Shore A Durometer.

In the belt length adjuster according to the present invention, a pinching angle β₁ between the outer frame portion 3c and the outer frame portion 8c and a pinching angle β₂ between the outer frame portion 5c and the outer frame portion 6c are both set to 30 to 60 degrees.

In the belt length adjuster according to the present invention, the belt B is made of a non-elastic synthetic resin.

In the belt length adjuster according to the present invention, recesses 9a are provided in inclined surfaces 4f at opposite ends of the pivot portion 4 of the first pinching frame 1, respectively and projections 9b are provided on both inner end faces in the opposite side frame portions 8a and 8b of the second pinching frame 2, respectively, allowing the recesses 9a and the projections 9b to fit together and thereby enabling the first pinching frame 1 and the second pinching frame 2 to be fixed to each other in a closed state when the crossing angle α is fully closed.

In the belt length adjuster according to the present invention, a plurality of pressing claws 10a are provided on an undersurface 5f of the outer frame portion 5c of the upper pinching frame body 5 of the first pinching frame 1, a plurality of pressing claws 10b are provided on a top face 3f of the outer frame portion 3c of the lower pinching frame body 3, a plurality of pressing claws 10a are provided on an undersurface 8f of the outer frame portion 8c of the upper pinching frame body 8 of the second pinching frame 2, and a plurality of pressing claws 10b are provided on a top face 6f of the outer frame portion 6c of the lower pinching frame body 6, to increase a pinching force on the pinched belt with the pressing claws 10a and the pressing claws 10b when the crossing angle α is fully closed.

Being configured as described above, the belt length adjuster according to the present invention makes it easy to carry out a belt length adjustment operation with eyewear or the like worn, has a simple structure, allows production costs to be reduced as well, and has the advantage of causing low fluid resistance during competitive swimming and the like when used for swimming goggles.

### BRIEF DESCRIPTION OF THE DRAWINGS

FIG. 1 is a perspective view of swimming goggles using a belt length adjuster according to the present invention, as viewed from the front;
FIG. 2 is a perspective view of the swimming goggles shown in FIG. 1, as viewed from the rear;
FIG. 3 is a plan view of the swimming goggles shown in FIG. 1;
FIG. 4 is a perspective view of swimming goggles using two belt length adjusters according to the present invention, as viewed from the rear;
FIG. 5 is a perspective view of the belt length adjuster according to the present invention;
FIG. 6 is an exploded perspective view of the belt length adjuster according to the present invention;
FIG. 7 is side view of the belt length adjuster shown in FIG. 5;
FIG. 8 is a sectional view of the belt length adjuster shown in FIG. 5;
FIG. 9 is a perspective view of the belt length adjuster shown in FIG. 5, with a crossing angle closed;
FIG. 10 is a side view of the belt length adjuster shown in FIG. 9;
FIG. 11 is a sectional view of the belt length adjuster shown in FIG. 9;
FIG. 12 is a perspective view of a belt length adjuster according to another embodiment of the present invention;
FIG. 13 is an exploded perspective view of the belt length adjuster shown in FIG. 12;
FIG. 14 is a perspective view of the belt length adjuster shown in FIG. 12, as viewed from another direction;
FIG. 15 is a side view of the belt length adjuster shown in FIG. 12;
FIG. 16 is a sectional view of the belt length adjuster shown in FIG. 12;
FIG. 17 is a perspective view of the belt length adjuster shown in FIG. 12, with a crossing angle closed;
FIG. 18 is a side view of the belt length adjuster shown in FIG. 17;
FIG. 19 is a sectional view of the belt length adjuster shown in FIG. 17;
FIGS. 20A and 20B show how a belt is passed through the belt length adjuster according to the present invention, where FIG. 20A is a sectional view showing a state in which the crossing angle of the belt length adjuster is fully opened and FIG. 20B is a sectional view showing a state in which the crossing angle of the belt length adjuster is fully closed;
FIG. 21 is a front view of an example of swimming goggles using a conventional belt length adjuster;
FIG. 22 is a partial sectional perspective view showing a state in which the belt length adjuster shown in FIG. 21 is removed from the swimming goggles;
FIG. 23 is a sectional view showing how a belt is passed through the belt length adjuster shown in FIG. 21;
FIG. 24 is a perspective view of swimming goggles using a conventional belt length adjuster of another structure;
FIG. 25 is an exploded perspective view showing a state in which the belt length adjuster shown in FIG. 24 is removed from the swimming goggles; and
FIG. 26 is a sectional view showing how a belt is passed through the belt length adjuster shown in FIG. 24.

### DETAILED DESCRIPTION OF THE PREFERRED EMBODIMENTS

A belt length adjuster according to an embodiment of the present invention will be described in detail below with reference to the drawings.

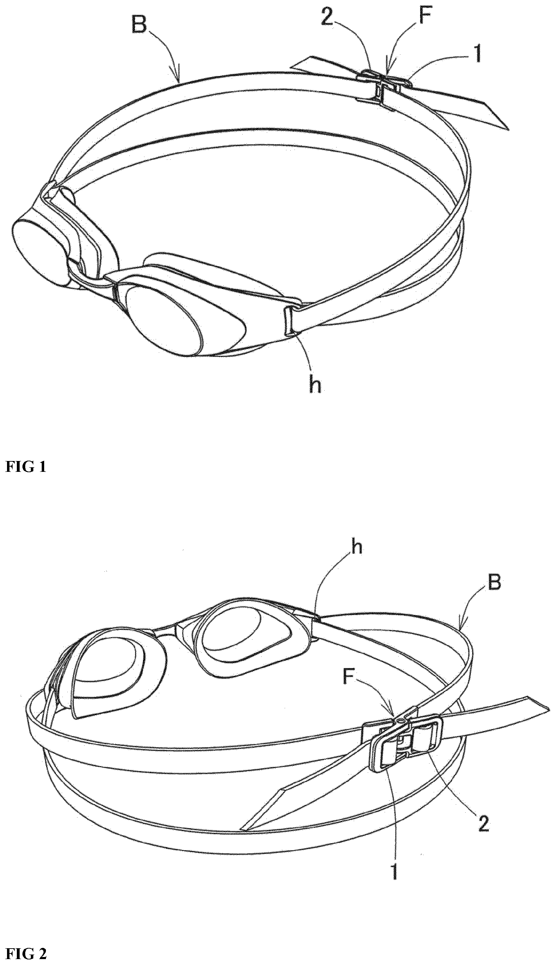

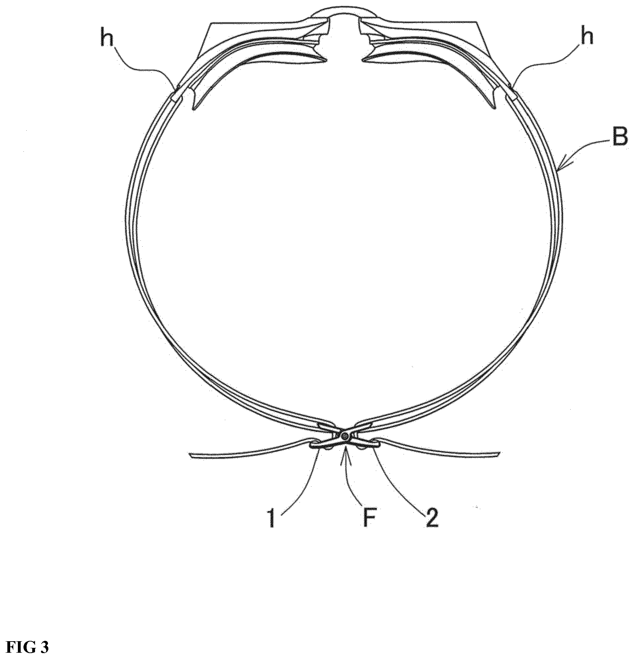

FIGS. 1 to 4 show swimming goggles equipped with a belt length adjuster according to the present invention, and a belt B used by a user to wear the swimming goggles is attached to opposite end portions of the lens frame. That is, belt insertion holes h are provided in the opposite end portions of the lens frame and the belt B inserted into the belt insertion holes h is attached to the opposite end portions of the lens frame with its length being adjusted freely by the belt length adjuster.

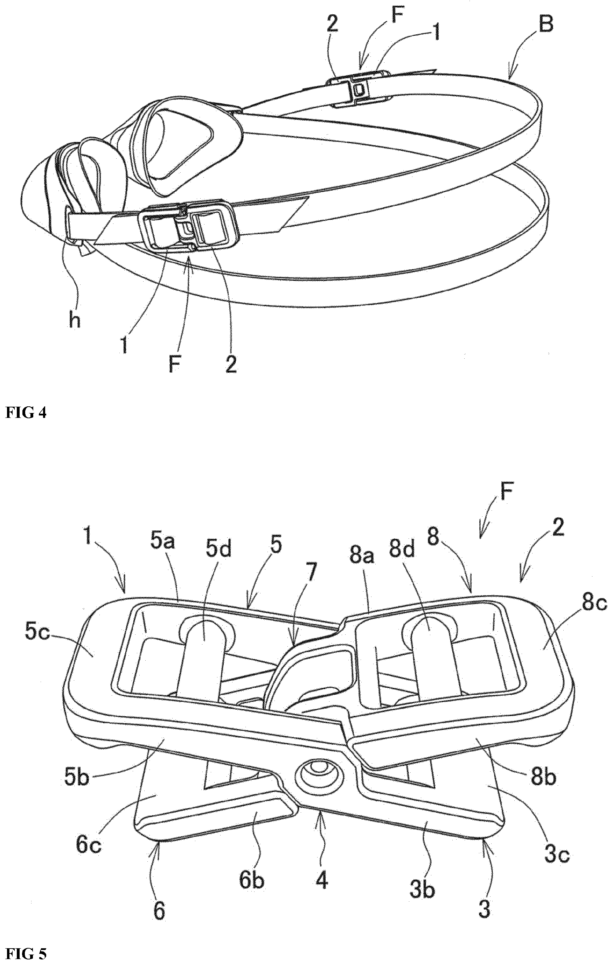

The belt length adjuster according to the present invention shown in FIGS. 1 to 3 is provided at one location of the belt B so as to be placed at the back of the head of the user wearing the swimming goggles. The belt length adjuster according to the present invention shown in FIG. 4 is provided at two locations of the belt B so as to be placed in both temporal regions of the head of the user wearing the swimming goggles.

In FIGS. 1 to 3, opposite ends of a single belt B are inserted into the belt insertion holes h in the opposite end portions of the lens frame, respectively, from inside outward and the belt length adjuster according to the present invention is attached to the opposite ends of the belt B with the opposite ends being doubled by turning back. Furthermore, in FIG. 4, the belt length adjuster according to the present invention is provided at two locations: opposite ends of a single belt B are inserted into the belt insertion holes h in the opposite end portions of the lens frame, respectively, from inside outward, tying the opposite ends of the belt B doubled by turning back, respectively, to opposite ends of another belt B overlaid on the first belt B. Note that both the belt B shown in FIGS. 1 to 3 and the belt B shown in FIG. 4 may be used singly by being fixed at opposite ends to the belt insertion holes h without being doubled by turning back after insertion into the belt insertion holes h.

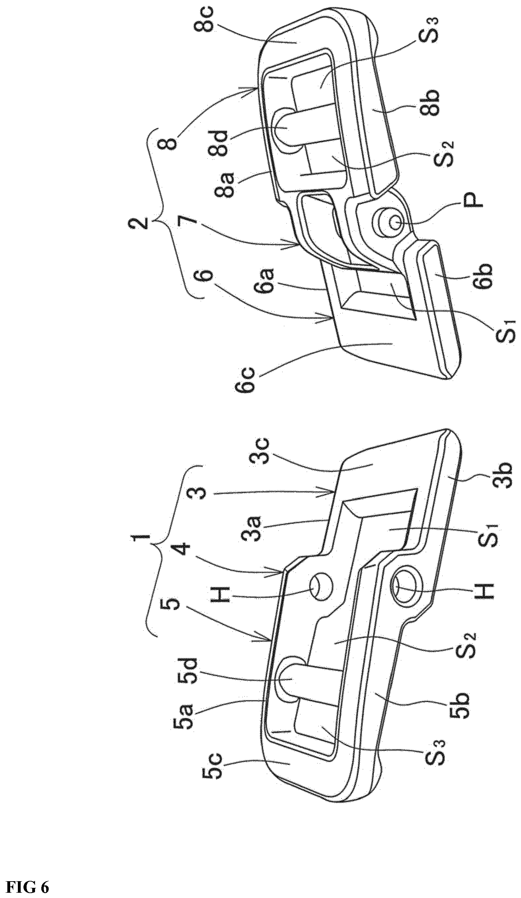

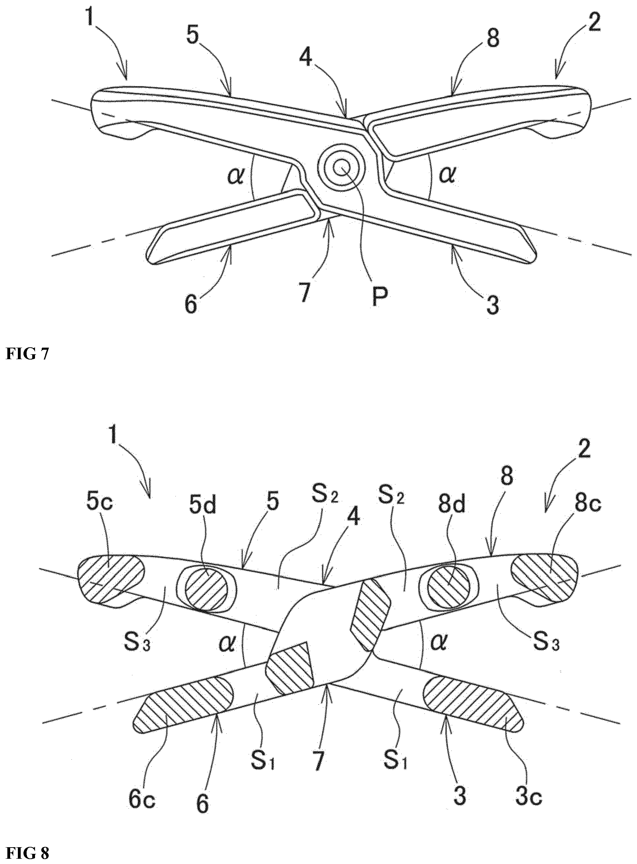

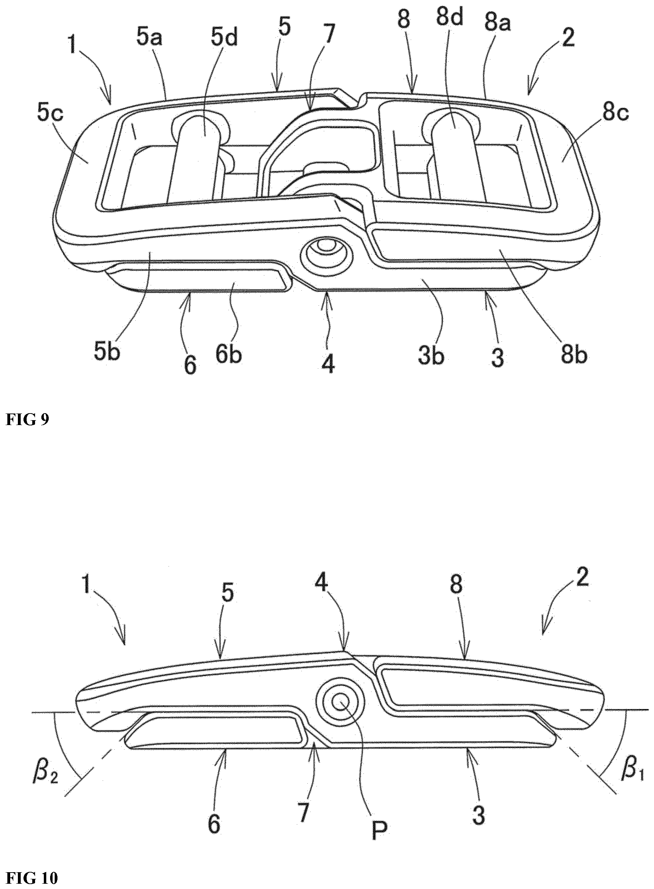

As shown in FIGS. 5 to 11, the belt length adjuster according to the present invention includes a belt pinching frame F, the belt pinching frame F including a first pinching frame 1 made of a hard resin such as polycarbonate, polyamide, or ABS resin, and a second pinching frame 2, where the first and second pinching frames 1 and 2 are pivotably supported by intersecting each other and allowed to pivot freely and a crossing angle α between the first and second pinching frames 1 and 2 are allowed to be opened and closed. A first end of a belt B is inserted into a first end of the belt pinching frame F and a second end of the belt B is inserted into a second end of the belt pinching frame F, the first end and the second end of the belt B are pinched between the first and second pinching frames 1 and 2 when the crossing angle α is fully closed, and the first end and the second end of the belt B are released from pinching when the crossing angle α is fully opened.

The crossing angle α can be set to 10 to 90 degrees, but to fully open the crossing angle α, the first pinching frame 1 and second pinching frame 2 are picked up with fingers or the opposite ends of the belt B are picked up. Thus, considering operability of the first and second pinching frames 1 and 2, preferably the crossing angle α is set to 20 to 40 degrees, and more preferably to about 30 degrees. When the crossing angle α becomes smaller than 10 degrees, the first pinching frame 1 and second pinching frame 2 are difficult to pick up in loosening the belt B and the belt B becomes less liable to loosen because friction remains strong. Conversely, when the crossing angle α becomes larger than 90 degrees, finger forces become prone to be exerted in loosening the belt B, which is liable to cause breakage.

The first pinching frame 1 includes a lower pinching frame body 3 on one side and an upper pinching frame body 5 on another side across a pivot portion 4, and has a substantially rectangular shape as a whole in planner view. The lower pinching frame body 3 includes opposite side frame portions 3a and 3b and an outer frame portion 3c, and a first insertion space S₁ for a belt B is provided on an inner side of the outer frame portion 3c. The upper pinching frame body 5 includes opposite side frame portions 5a and 5b, an outer frame portion 5c, and a middle frame portion 5d, a second insertion space S₂ for the belt B is provided on an inner side of the middle frame portion 5d, and a third insertion space S₃ for the belt B is provided between the outer frame portion 5c and the middle frame portion 5d. The pivot portion 4 can be either a recess or a projection. If a pivot portion 7 of the second pinching frame 2 is configured as a projection such as described later, the pivot portion 4 can be a recess such as through-holes H provided in the first pinching frame 1 as illustrated. Conversely, if the pivot portion 7 of the second pinching frame 2 is configured as such a recess, the pivot portion 4 can be a projection. Furthermore, because the belt B slides along the outer frame portion 3c and middle frame portion 5d in adjusting the belt length as described later, sliding surfaces of the outer frame portion 3c and middle frame portion 5d with respect to the belt B are rounded to enable smooth sliding. Note that respective slit widths of the first insertion space S₁, second insertion space S₂, and third insertion space S₃ are set sufficiently larger than a thickness of the belt B described later.

The second pinching frame 2 includes a lower pinching frame body 6 on one side and an upper pinching frame body 8 on another side across a pivot portion 7, and has a substantially rectangular shape as a whole in planner view. The lower pinching frame body 6 includes opposite side frame portions 6a and 6b and an outer frame portion 6c and the first insertion space S₁ for the belt B is provided on an inner side of the outer frame portion 6c. The upper pinching frame body 8 includes opposite side frame portions 8a and 8b, an outer frame portion 8c, and a middle frame portion 8d, the second insertion space S₂ for the belt B is provided on an inner side of the middle frame portion 8d, and the third insertion space S₃ for the belt B is provided between the outer frame portion 8c and the middle frame portion 8d. The pivot portion 7 can be either a recess or a projection. If the pivot portion 4 of the first pinching frame 1 is configured as a recess such as described above, the pivot portion 7 is configured as a projection such as projections P provided on the second pinching frame 2 as illustrated. Conversely, if the pivot portion 4 of the first pinching frame 1 is configured as such a projection, the pivot portion 7 can be a recess. Furthermore, because the belt B slides along the outer frame portion 6c and middle frame portion 8d in adjusting the belt length as in the above case, sliding surfaces of the outer frame portion 6c and middle frame portion 8d with respect to the belt B are rounded to enable smooth sliding. Note that similarly, respective slit widths of the first insertion space S₁, second insertion space S₂, and third insertion space S₃ in the second pinching frame 2 are set sufficiently larger than the thickness of the belt B described later.

When used for swimming goggles, the belt B is made of an elastic synthetic resin or synthetic rubber, such as elastomer or silicone rubber, with an elasticity of 25 to 65 Shore A Durometer, and is preferably 1.2 to 2.4 mm in thickness, but may be made of a non-elastic synthetic resin when used for a belt of clothing or accessories. Note that the first end of the belt B is pinched between the outer frame portion 3c of the first pinching frame 1 and the outer frame portion 8c of the second pinching frame 2, and the second end of the belt B is pinched between the outer frame portion 5c of the first pinching frame 1 and the outer frame portion 6c of the second pinching frame 2. In this case, if the belt B is made of an elastic synthetic resin or synthetic rubber with an elasticity of 25 to 65 Shore A Durometer, a pinching angle β₁ between the outer frame portion 3c and the outer frame portion 8c and a pinching angle β₂ between the outer frame portion 5c and the outer frame portion 6c are preferably both set to 30 to 60 degrees, and more preferably to about 45 degrees, but may be set to an angle according to hardness, a friction coefficient, and the like of the belt B used.

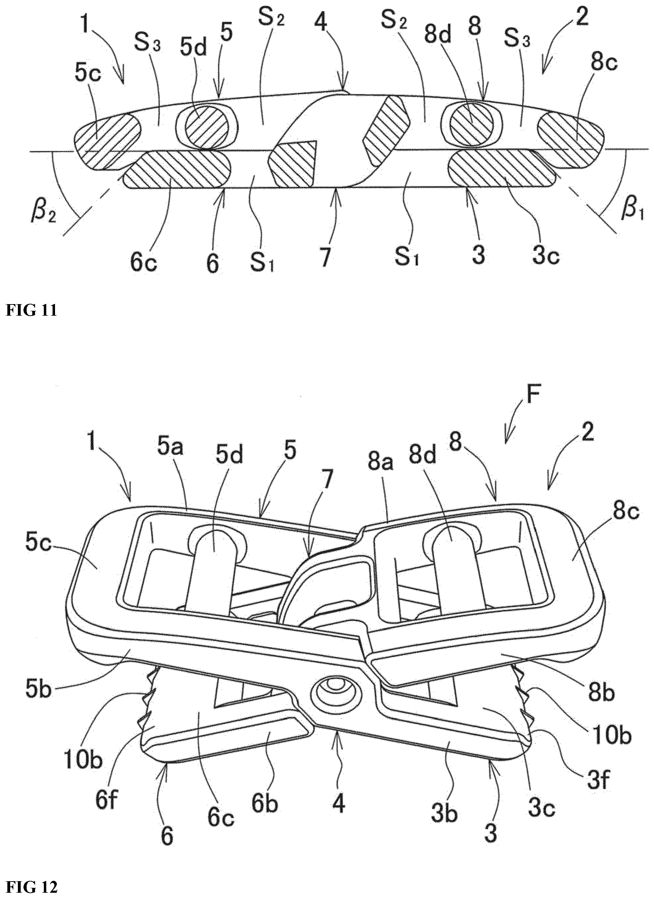

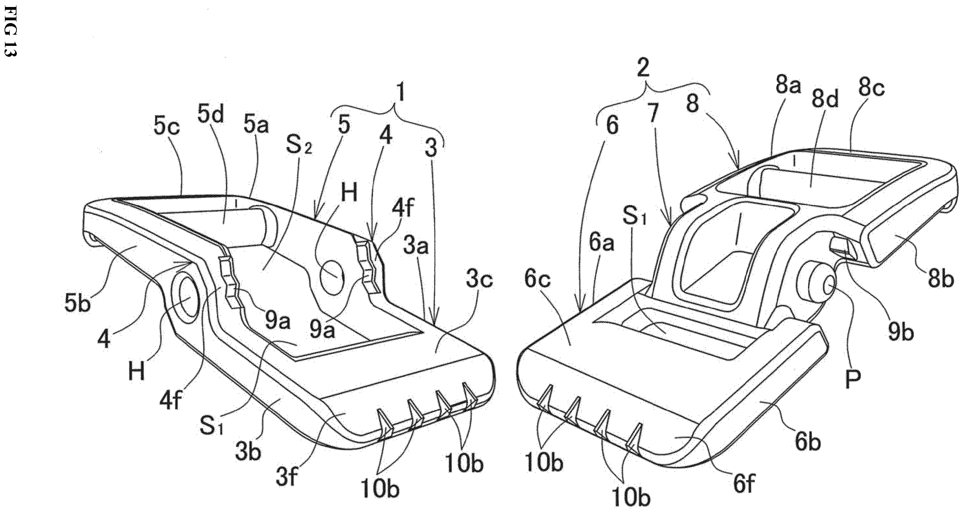

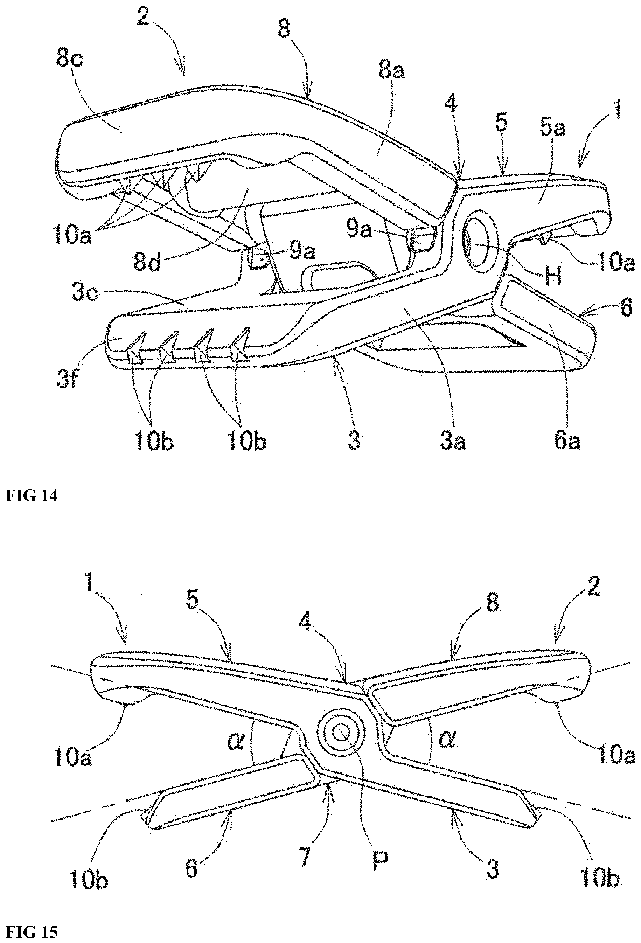

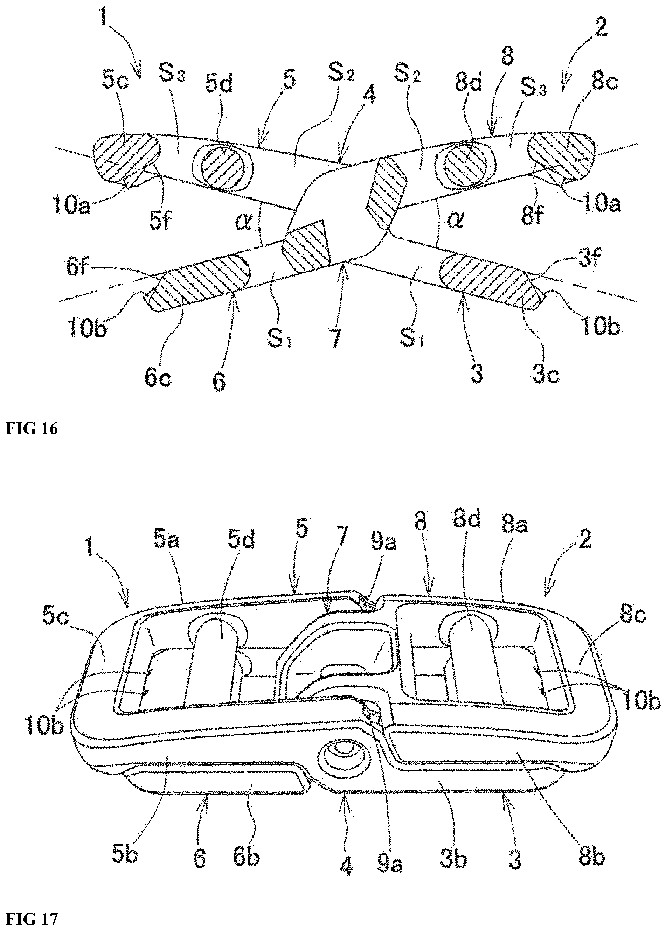

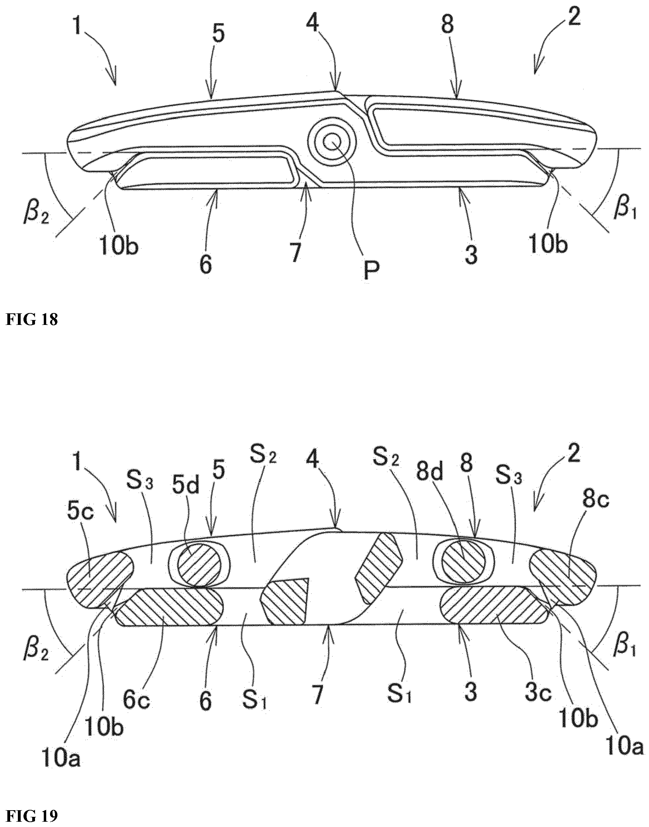

Note that when the belt B is made of a non-elastic synthetic resin, structures such as shown in FIGS. 12 to 19 may be added to the belt length adjuster according to the present invention.

In the belt length adjuster described above, recesses 9a are provided in inclined surfaces 4f at opposite ends of the pivot portion 4 of the first pinching frame 1, respectively, and projections 9b are provided on both inner end faces in the opposite side frame portions 8a and 8b of the second pinching frame 2, respectively, allowing the recesses 9a and the projections 9b to fit together and thereby enabling the first pinching frame 1 and the second pinching frame 2 to be fixed to each other in a closed state when the crossing angle α is fully closed.

Furthermore, in the belt length adjuster described above, a plurality of pressing claws 10a are provided on an undersurface 5f of the outer frame portion 5c of the upper pinching frame body 5 of the first pinching frame 1, a plurality of pressing claws 10b are provided on a top face 3f of the outer frame portion 3c of the lower pinching frame body 3, a plurality of pressing claws 10a are provided on an undersurface 8f of the outer frame portion 8c of the upper pinching frame body 8 of the second pinching frame 2, and a plurality of pressing claws 10b are provided on a top face 6f of the outer frame portion 6c of the lower pinching frame body 6, to increase a pinching force on the pinched belt with the pressing claws 10a and the pressing claws 10b when the crossing angle α is fully closed.

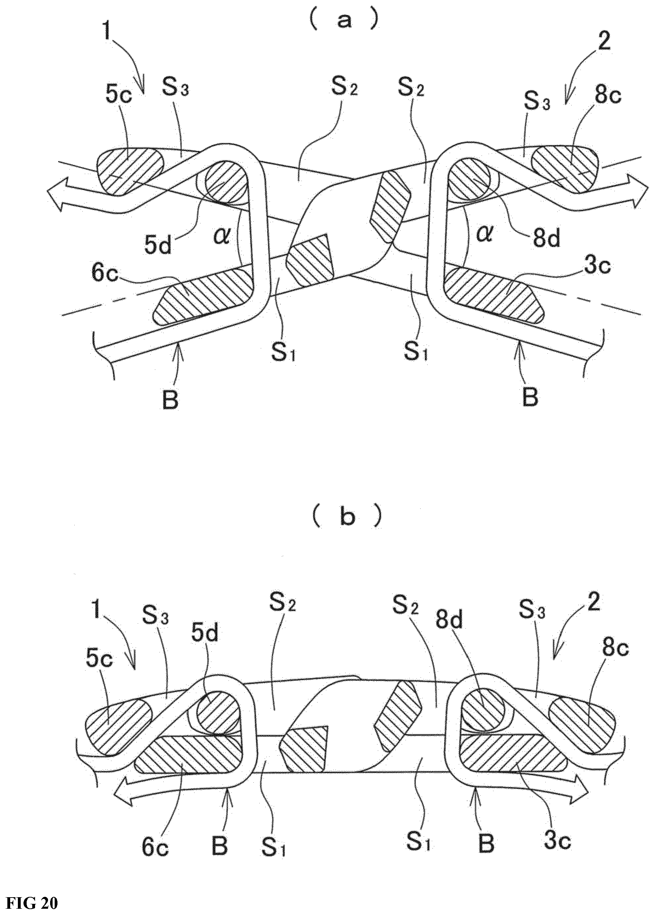

In the belt length adjuster according to the present invention configured as described above, the first end of the belt B is inserted into the first end of the belt pinching frame F and the second end of the belt B is inserted into the second end of the belt pinching frame F, and the first end and second end of the belt B are pinched between the first and second pinching frames 1 and 2 and released from pinching in a manner shown in FIGS. 20A and 20B.

First, as shown in FIG. 20A, the crossing angle α between the first pinching frame 1 and the second pinching frame 2 is fully opened, the first end of the belt B is inserted into the first insertion space S₁ in the first pinching frame 1 from below the first insertion space S₁, inserted into the second insertion space S₂ in the second pinching frame 2 from below the second insertion space S₂, and further inserted into the third insertion space S₃ in the second pinching frame 2 from above the third insertion space S₃, and pulled out of the third insertion space S₃ downward.

Next, the second end of the belt B is inserted into the first insertion space S₁ in the second pinching frame 2 from below the first insertion space S₁, inserted into the second insertion space S₂ in the first pinching frame 1 from below the second insertion space S₂, and further inserted into the third insertion space S₃ in the first pinching frame 1 from above the third insertion space S₃, and pulled out of the third insertion space S₃ downward.

Next, as shown in FIG. 20B, when the crossing angle α between the first pinching frame 1 and the second pinching frame 2 is fully closed, the first end of the belt B is pinched between the outer frame portion 3c of the first pinching frame 1 and the outer frame portion 8c of the second pinching frame 2 while the second end of the belt B is pinched between the outer frame portion 5c of the first pinching frame 1 and the outer frame portion 6c of the second pinching frame 2.

A repeated belt load test (JIS S7301 7.6) was conducted on the belt of swimming goggles equipped with the belt length adjuster according to the present invention configured as described above and it was confirmed that the belt did not loosen with the swimming goggles put on.

### [Test procedure]

The swimming goggles were put on a steel pipe (approximately 200 mm in diameter), the belt portion was pulled repeatedly with a frequency of 60 times per minute under a load of 19.6 N for 20 minutes, and the belt was checked for anything unusual. The belt was tested after being immersed in water for one hour. The acceptability criteria were that there was no belt displacement of 10 mm or more on one side and there was no cut.

Regarding the configuration of the belt length adjuster according to the present invention, the crossing angle α between the first and second pinching frames 1 and 2 was 30 degrees, the pinching angle β₁ between the outer frame portion 3c and the outer frame portion 8c and the pinching angle β₂ between the outer frame portion 5c and the outer frame portion 6c were both 45 degrees, and the sliding surfaces of the outer frame portion 3c and middle frame portion 5d with respect to the belt B as well as the sliding surfaces of the outer frame portion 6c and middle frame portion 8d with respect to the belt B were rounded. The belt B had a hardness of 50 Shore A Durometer, a thickness of 1.3 mm, and a width of 7 mm. With this configuration, the repeated belt load test revealed no displacement or cut of the belt B.

On the belt of the swimming goggles equipped with the belt length adjuster according to the present invention configured as described above, the belt length is adjusted as follows.

First, when the crossing angle α between the first pinching frame 1 and the second pinching frame 2 is fully closed as shown in FIG. 20B, the belt length adjuster according to the present invention is very compact and is placed at the back of the head, or in both temporal regions, of the user if the swimming goggles are worn in this state.

Thus, when the belt B of the swimming goggles the user is wearing is loose, if the user pulls the opposite ends of the belt B in a direction away from the head by gripping each end with fingers in the state shown in FIG. 20B, the crossing angle α between the first pinching frame 1 and the second pinching frame 2 is fully opened as shown in FIG. 20A.

Next, with the crossing angle α between the first pinching frame 1 and the second pinching frame 2 kept fully open, when the opposite ends of the belt B are pulled in a direction away from each other (in a right-left direction), the belt B contracts on the side of the first end by sliding along the middle frame portion 5d of the first pinching frame 1 and the outer frame portion 6c of the second pinching frame 2 and contracts on the side of the second end by sliding along the middle frame portion 8d of the second pinching frame 2 and the outer frame portion 3c of the first pinching frame 1, tightening the belt B. When the forces pulling the opposite ends of the belt B are relaxed, tension from the side of the swimming goggles grows larger and the middle frame portion 5d of the first pinching frame 1 and the middle frame portion 8d of the second pinching frame 2 are pulled toward the head by an elastic force of the belt B, thereby closing the crossing angle α as shown in FIG. 20B. Then, since the first end of the belt B is pinched between the outer frame portion 3c of the first pinching frame 1 and the outer frame portion 8c of the second pinching frame 2 while the second end of the belt B is pinched between the outer frame portion 5c of the first pinching frame 1 and the outer frame portion 6c of the second pinching frame 2, even if the fingers are taken off the opposite ends of the belt B, the belt remains fixed without loosening.

Also, when the belt B of the swimming goggles the user is wearing is tight, if the user pulls up the outer frame portion 5c of the first pinching frame 1 and the outer frame portion 8c of the second pinching frame 2 in a direction away from the head by gripping each of the outer frame portions with fingers in the state shown in FIG. 20B, the crossing angle α between the first pinching frame 1 and the second pinching frame 2 is fully opened as shown in FIG. 20A.

Next, with the crossing angle α between the first pinching frame 1 and the second pinching frame 2 kept fully open, if the user further pulls up the first pinching frame 1 and second pinching frame 2 gripped with the fingers in a direction away from the head, the belt B stretches on the side of the first end by sliding along the middle frame portion 5d of the first pinching frame 1 and the outer frame portion 6c of the second pinching frame 2 and stretches also on the side of the second end by sliding along the middle frame portion 5d of the first pinching frame 1 and the outer frame portion 6c of the second pinching frame 2, loosening the belt. When the forces pulling up the first pinching frame 1 and second pinching frame 2 are relaxed, the tension from the side of the swimming goggles grows larger as in the above case, and the middle frame portion 5d of the first pinching frame 1 and the middle frame portion 8d of the second pinching frame 2 are pulled toward the head by an elastic force of the belt B, thereby closing the crossing angle α as shown in FIG. 20B. Then, since the first end of the belt B is pinched between the outer frame portion 3c of the first pinching frame 1 and the outer frame portion 8c of the second pinching frame 2 while the second end of the belt B is pinched between the outer frame portion 5c of the first pinching frame 1 and the outer frame portion 6c of the second pinching frame 2, even if the fingers are taken off the opposite ends of the belt B, the belt remains fixed without loosening.

As described above, the belt length adjuster according to the present invention makes it easy to carry out a belt length adjustment operation with eyewear or the like worn, has a simple structure, allows production costs to be reduced as well, and has a great advantage of causing low fluid resistance during competitive swimming and the like when used for swimming goggles.

## Claims

1. A belt length adjuster comprising a belt pinching frame (F), the belt pinching frame (F) comprising a first pinching frame (1) and a second pinching frame (2) pivotally intersecting each other, a crossing angle (α) between the first and second pinching frames (1 and 2) being able to be opened and closed, wherein in use, the belt length adjuster is configured so that a first end of a belt (B) can be inserted into a first end of the belt pinching frame (F) and a second end of the belt (B) can be inserted into a second end of the belt pinching frame (F), and wherein in use, the belt length adjuster is configured so that the first end and the second end of the belt (B) can be pinched between the first and second pinching frames (1 and 2) when the crossing angle (α) is fully closed, and the first end and the second end of the belt (B) can be released from pinching when the crossing angle (α) is fully opened;
wherein: the first pinching frame (1) includes a lower pinching frame body (3) on one side and an upper pinching frame body (5) on another side across a pivot portion (4); the lower pinching frame body (3) includes opposite side frame portions (3a and 3b) and an outer frame portion (3c); a first insertion space (S₁) for a belt (B) is provided on an inner side of the outer frame portion (3c); the upper pinching frame body (5) includes opposite side frame portions (5a and 5b), an outer frame portion (5c), and a middle frame portion (5d); a second insertion space (S₂) for the belt (B) is provided on an inner side of the middle frame portion (5d); a third insertion space (S₃) for the belt (B) is provided between the outer frame portion (5c) and the middle frame portion (5d); the second pinching frame (2) includes a lower pinching frame body (6) on one side and an upper pinching frame body (8) on another side across a pivot portion (7); the lower pinching frame body (6) includes opposite side frame portions (6a and 6b) and an outer frame portion (6c); the first insertion space (S₁) for the belt (B) is provided on an inner side of the outer frame portion (6c); the upper pinching frame body (8) includes opposite side frame portions (8a and 8b), an outer frame portion (8c), and a middle frame portion (8d); the second insertion space (S₂) for the belt (B) is provided on an inner side of the middle frame portion (8d); and the third insertion space (S₃) for the belt (B) is provided between the outer frame portion (8c) and the middle frame portion (8d).

2. The belt length adjuster according to claim 1, wherein the first end of the belt (B) can be pinched between the outer frame portion (3c) of the first pinching frame (1) and the outer frame portion (8c) of the second pinching frame (2); and the second end of the belt (B) can be pinched between the outer frame portion (5c) of the first pinching frame (1) and the outer frame portion (6c) of the second pinching frame (2).

3. The belt length adjuster according to claim 1 or claim 2, wherein the crossing angle (α) is set to 10 to 90 degrees.

4. The belt length adjuster according to any one of claims 1 to 3, wherein the belt (B) is made of an elastic synthetic resin or synthetic rubber with an elasticity of 25 to 65 Shore A Durometer.

5. The belt length adjuster according to any one of claims 1 to 4, wherein a pinching angle (β₁) between the outer frame portion (3c) and the outer frame portion (8c) and a pinching angle (β₂) between the outer frame portion (5c) and the outer frame portion (6c) are both set to 30 to 60 degrees.

6. The belt length adjuster according to any one of claims 1 to 5, wherein the belt (B) is made of a non-elastic synthetic resin.

7. The belt length adjuster according to claim 6, wherein recesses (9a) are provided in inclined surfaces (4f) at opposite ends of the pivot portion (4) of the first pinching frame (1), respectively, and projections (9b) are provided on both inner end faces in the opposite side frame portions (8a and 8b) of the second pinching frame (2), respectively, allowing the recesses (9a) and the projections (9b) to fit together and thereby enabling the first pinching frame (1) and the second pinching frame (2) to be fixed to each other in a closed state when the crossing angle (α) is fully closed.

8. The belt length adjuster according to claim 6 or 7, wherein a plurality of pressing claws (10a) are provided on an undersurface (5f) of the outer frame portion (5c) of the upper pinching frame body (5) of the first pinching frame (1), a plurality of pressing claws (10b) are provided on a top face (3f) of the outer frame portion (3c) of the lower pinching frame body (3), a plurality of pressing claws (10a) are provided on an undersurface (8f) of the outer frame portion (8c) of the upper pinching frame body (8) of the second pinching frame (2), and a plurality of pressing claws (10b) are provided on a top face (6f) of the outer frame portion (6c) of the lower pinching frame body (6), to increase a pinching force on the pinched belt with the pressing claws (10a) and the pressing claws (10b) when the crossing angle (α) is fully closed.

## Patentansprüche

1. Gurtlängenanpassungsvorrichtung, die einen Gurtklemmrahmen (F) umfasst, wobei der Gurtklemmrahmen (F) einen ersten Klemmrahmen (1) und einen zweiten Klemmrahmen (2) umfasst, die sich drehgelenkig überschneiden, wobei ein Kreuzungswinkel (α) zwischen dem ersten und zweiten Klemmrahmen (1 und 2) geöffnet und geschlossen werden kann, wobei bei Benutzung die Gurtlängenanpassungsvorrichtung so konfiguriert ist, dass ein erstes Ende eines Gurtes (B) in ein erstes Ende des Gurtklemmrahmens (F) eingesetzt werden kann und ein zweites Ende des Gurtes (B) in ein zweites Ende des Gurtklemmrahmens (F) eingesetzt werden kann und wobei bei Benutzung die Gurtlängenanpassungsvorrichtung so konfiguriert ist, dass das erste Ende und das zweite Ende des Gurtes (B) zwischen dem ersten und zweiten Klemmrahmen (1 und 2) eingeklemmt werden können, wenn der Kreuzungswinkel (α) vollständig geschlossen ist, und das erste Ende und das zweite Ende des Gurtes (B) vom Einklemmen gelöst werden können, wenn der Kreuzungswinkel (α) vollständig geöffnet ist;
wobei: der erste Klemmrahmen (1) einen unteren Klemmrahmenkörper (3) auf einer Seite und einen oberen Klemmrahmenkörper (5) auf einer anderen Seite über einen Drehteil (4) aufweist; der untere Klemmrahmenkörper (3) gegenüberliegende Seitenrahmenteile (3a und 3b) und einen äußeren Rahmenteil (3c) aufweist; ein erster Einführungsraum (S₁) für einen Gurt (B) auf einer inneren Seite des äußeren Rahmenteils (3c) bereitgestellt wird; der obere Klemmrahmenkörper (5) gegenüberliegende Seitenrahmenteile (5a und 5b), einen äußeren Rahmenteil (5c) und einen mittleren Rahmenteil (5d) aufweist; ein zweiter Einführungsraum (S₂) für den Gurt (B) auf einer inneren Seite des mittleren Rahmenteils (5d) bereitgestellt wird; ein dritter Einführungsraum (S₃) für den Gurt (B) zwischen dem äußeren Rahmenteil (5c) und dem mittleren Rahmenteil (5d) bereitgestellt wird; der zweite Klemmrahmen (2) einen unteren Klemmrahmenkörper (6) auf einer Seite und einen oberen Klemmrahmenkörper (8) auf einer anderen Seite über einen Drehteil (7) aufweist; der untere Klemmrahmenkörper (6) gegenüberliegende Seitenrahmenteile (6a und 6b) und einen äußeren Rahmenteil (6c) aufweist; der erste Einführungsraum (S₁) für den Gurt (B) an einer inneren Seite des äußeren Rahmenteils (6c) bereitgestellt wird; der obere Klemmrahmenkörper (8) gegenüberliegende Seitenrahmenteile (8a und 8b), einen äußeren Rahmenteil (8c) und einen mittleren Rahmenteil (8d) aufweist; der zweite Einführungsraum (S₂) für den Gurt (B) an einer inneren Seite des mittleren Rahmenteils (8d) bereitgestellt wird; und der dritte Einführungsraum (S₃) für den Gurt (B) zwischen dem äußeren Rahmenteil (8c) und dem mittleren Rahmenteil (8d) bereitgestellt wird.

2. Gurtlängenanpassungsvorrichtung nach Anspruch 1, wobei das erste Ende des Gurtes (B) zwischen dem äußeren Rahmenteil (3c) des ersten Klemmrahmens (1) und dem äußeren Rahmenteil (8c) des zweiten Klemmrahmens (2) eingeklemmt werden kann und das zweite Ende des Gurtes (B) zwischen dem äußeren Rahmenteil (5c) des ersten Klemmrahmens (1) und dem äußeren Rahmenteil (6c) des zweiten Klemmrahmens (2) eingeklemmt werden kann.

3. Gurtlängenanpassungsvorrichtung nach Anspruch 1 oder Anspruch 2, wobei der Kreuzungswinkel (α) auf 10 bis 90 Grad eingestellt ist.

4. Gurtlängenanpassungsvorrichtung nach einem der Ansprüche 1 bis 3, wobei der Gurt (B) aus einem elastischen Kunstharz oder synthetischem Gummi mit einer Elastizität von 25 bis 65 Shore A Durometer besteht.

5. Gurtlängenanpassungsvorrichtung nach einem der Ansprüche 1 bis 4, wobei ein Klemmwinkel (ß₁) zwischen dem äußeren Rahmenteil (3c) und dem äußeren Rahmenteil (8c) und ein Klemmwinkel (ß₂) zwischen dem äußeren Rahmenteil (5c) und dem äußeren Rahmenteil (6c) beide auf 30 bis 60 Grad eingestellt sind.

6. Gurtlängenanpassungsvorrichtung nach einem der Ansprüche 1 bis 5, wobei der Gurt (B) aus einem nicht-elastischen Kunstharz besteht.

7. Gurtlängenanpassungsvorrichtung nach Anspruch 6, wobei Aussparungen (9a) in schrägen Flächen (4f) an gegenüberliegenden Enden des Drehteils (4) des ersten Klemmrahmens (1) bzw. Vorsprünge (9b) an beiden inneren Stirnflächen in den gegenüberliegenden Seitenrahmenteilen (8a und 8b) des zweiten Klemmrahmens (2) bereitgestellt werden, die es ermöglichen, dass die Aussparungen (9a) und die Vorsprünge (9b) zusammenpassen und dadurch den ersten Klemmrahmen (1) und den zweiten Klemmrahmen (2) miteinander in einem geschlossenen Zustand fixieren können, wenn der Kreuzungswinkel (α) vollständig geschlossen ist.

8. Gurtlängenanpassungsvorrichtung nach Anspruch 6 oder 7, wobei eine Vielzahl von Druckkrallen (10a) an einer unteren Fläche (5f) des äußeren Rahmenteils (5c) des oberen Klemmrahmenkörpers (5) des ersten Klemmrahmens (1) bereitgestellt wird, eine Vielzahl von Druckkrallen (10b) an einer oberen Fläche (3f) des äußeren Rahmenteils (3c) des unteren Klemmrahmenkörpers (3) bereitgestellt wird, eine Vielzahl von Druckkrallen (10a) an einer unteren Fläche (8f) des äußeren Rahmenteils (8c) des oberen Klemmrahmenkörpers (8) des zweiten Klemmrahmens (2) bereitgestellt wird und eine Vielzahl von Druckkrallen (10b) an einer oberen Fläche (6f) des äußeren Rahmenteils (6c) des unteren Klemmrahmenkörpers (6) bereitgestellt wird, um eine Klemmkraft auf den eingeklemmten Gurt mit den Druckkrallen (10a) und den Druckkrallen (10b) zu erhöhen, wenn der Kreuzungswinkel (α) vollständig geschlossen ist.

## Revendications

1. Régleur de longueur de courroie comportant un cadre de pincement de courroie (F), le cadre de pincement de courroie (F) comportant un premier cadre de pincement (1) et un second cadre de pincement (1) se croisant de manière pivotante, un angle d'intersection (α) entre le premier et le second cadre de pincement (1 et 2) pouvant être ouvert et fermé, où lors de l'utilisation, le régleur de longueur de courroie est configuré de manière à ce qu'une première extrémité d'une courroie (B) puisse être insérée dans une première extrémité du cadre de pincement de courroie (F) et une seconde extrémité de la courroie (B) puisse être insérée dans une seconde extrémité du cadre de pincement de courroie (F), et où, lors de l'utilisation, le régleur de longueur de courroie est configuré de manière à ce que la première extrémité et la seconde extrémité de la courroie (B) puissent être pincées entre le premier et le second cadre de pincement (1 et 2) quand l'angle d'intersection (α) est complètement fermé, et la première extrémité et la seconde extrémité de la courroie (B) puissent être relâchées quand l'angle d'intersection (α) est complètement ouvert ;
où : le premier cadre de pincement (1) comprend un corps de cadre de pincement inférieur (3) sur un côté et un corps de cadre de pincement supérieur (5) sur un autre côté de part et d'autre d'une partie de pivotement (4) ; le corps de cadre de pincement inférieur (3) comprend des parties de cadre latérales opposées (3a et 3b) et une partie de cadre extérieure (3c) ; un premier espace d'insertion (S₁) pour une courroie (B) est prévu sur un côté intérieur de la partie de cadre extérieure (3c) ; le corps de cadre de pincement supérieur (5) comprend des parties de cadre latérales opposées (5a et 5b), une partie de cadre extérieure (5c) et une partie de cadre centrale (5d) ; un second espace d'insertion (S₂) pour la courroie (B) est prévu sur un côté intérieur de la partie de cadre centrale (5d) ; un troisième espace d'insertion (S₃) pour la courroie (B) est prévu entre la partie de cadre extérieure (5c) et la partie de cadre centrale (5d) ; le second cadre de pincement (2) comprend un corps de cadre de pincement inférieur (6) sur un côté et un corps de cadre de pincement supérieur (8) sur un autre côté de part et d'autre d'une partie de pivotement (7) ; le corps de cadre de pincement inférieur (6) comprend des parties de cadre latérales opposées (6a et 6b) et une partie de cadre extérieure (6c) ; le premier espace d'insertion (S₁) pour une courroie (B) est prévu sur un côté intérieur de la partie de cadre extérieure (6c) ; le corps de cadre de pincement supérieur (8) comprend des parties de cadre latérales opposées (8a et 8b), une partie de cadre extérieure (8c) et une partie de cadre centrale (8d) ; le second espace d'insertion (S₂) pour la courroie (B) est prévu sur un côté intérieur de la partie de cadre centrale (8d) ; et le troisième espace d'insertion (S₃) pour la courroie (B) est prévu entre la partie de cadre extérieure (8c) et la partie de cadre centrale (8d).

2. Régleur de longueur de courroie selon la revendication 1, où la première extrémité de la courroie (B) peut être pincée entre la partie de cadre extérieure (3c) du premier cadre de pincement (1) et la partie de cadre extérieure (8c) du second cadre de pincement (2) ; et la seconde extrémité de la courroie (B) peut être pincée entre la partie de cadre extérieure (5c) du premier cadre de pincement (1) et la partie de cadre extérieure (6c) du second cadre de pincement (2).

3. Régleur de longueur de courroie selon la revendication 1 ou la revendication 2, où l'angle d'intersection (α) est de 10 à 90 degrés.

4. Régleur de longueur de courroie selon l'une quelconque des revendications 1 à 3, où la courroie (B) est composée d'une résine synthétique ou d'un caoutchouc synthétique élastiques ayant une élasticité de 25 à 65 au duromètre Shore A.

5. Régleur de longueur de courroie selon l'une quelconque des revendications 1 à 4, où un angle de pincement (β₁) entre la partie de cadre extérieure (3c) et la partie de cadre extérieure (8c) et un angle de pincement (β₂) entre la partie de cadre extérieure (5c) et la partie de cadre extérieure (6c) sont tous deux de 30 à 60 degrés.

6. Régleur de longueur de courroie selon l'une quelconque des revendications 1 à 5, où la courroie (B) est composée d'une résine synthétique non élastique.

7. Régleur de longueur de courroie selon la revendication 6, où des évidements (9a) sont respectivement prévus dans des surface inclinées (4f) à des extrémités opposées de la partie de pivotement (4) du premier cadre de pincement (1), et des saillies (9b) sont respectivement prévues sur les deux faces d'extrémité intérieures dans les parties de cadre latérales opposées (8a et 8b) du second cadre de pincement (2), permettant aux évidements (9a) et aux saillies (9b) de s'assembler et permettant ainsi au premier cadre de pincement (1) et au second cadre de pincement (2) d'être fixés l'un à l'autre à l'état fermé quand l'angle d'intersection (α) est complètement fermé.

8. Régleur de longueur de courroie selon la revendication 6 ou 7, où une pluralité de griffes pressantes (10a) sont prévues sur une surface inférieure (5f) de la partie de cadre extérieure (5c) du corps de cadre de pincement supérieur (5) du premier cadre de pincement (1), une pluralité de griffes pressantes (10b) sont prévues sur une surface supérieure (3f) de la partie de cadre extérieure (3c) du corps de cadre de pincement inférieur (3), une pluralité de griffes pressantes (10a) sont prévues sur une surface inférieure (8f) de la partie de cadre extérieure (8c) du corps de cadre de pincement supérieur (8) du second cadre de pincement (2), et une pluralité de griffes pressantes (10b) sont prévues sur une surface supérieure (6f) de la partie de cadre extérieure (6c) du corps de cadre de pincement inférieur (6), afin d'augmenter une force de pincement sur la courroie pincée au moyen des pinces pressantes (10a) et des pinces pressantes (10b) quand l'ange d'intersection (α) est complètement fermé.
